# DEMANDE DE BREVET EUROPEEN

(11) **EP 1 543 787 A1**
(43) Date de publication de la demande: **22.06.2005**
(21) Numéro de dépôt: 04356194.3
(22) Date de dépôt: 14.12.2004
(51) Int. Cl.: A61B 17/68, A61B 17/80

(54) **Dispositif implantable d'ostéosynthèse**

(30) Priorité: 15.12.2003 FR 0314674
(71) Demandeur: Biomet Merck France, 26903 Valence Cedex (FR); Augoyard, Marc, 69160 Tassin la Demi Lune (FR); Peyrot, Jacques, 69160 Tassin la Demi Lune (FR); Maestro, Michel, 06200 Nice (FR); Benichou, Michel, 34070 Montpellier (FR); Ragusa, Mathieu, 38240 Meylan (FR); Meusnier, Tristan, 42000 St Etienne (FR)
(72) Inventeur: Augoyard, Marc, 69160 Tassin la Demi Lune (FR); Peyrot, Jacques, 69160 Tassin la Demi Lune (FR); Maestro, Michel, 06200 Nice (FR); Benichou, Michel, 34070 Montpellier (FR); Ragusa, Mathieu, 38240 Meylan (FR); Meusnier, Tristan, 42000 St Etienne (FR); Brunnarius, Yann, 07130 St Peray (FR); de Witte, Gérard, 26300 Chateauneuf sur Isere (FR)
(74) Mandataire: Thibault, Jean-Marc

(57) **Abrégé**

Selon l'invention, le dispositif implantable d'ostéosynthèse comprend un corps allongé pourvu, au moins au niveau de ses deux extrémités, de moyens d'ancrage (**3**) dans un os ou partie d'os. Le dispositif est caractérisé en ce que le corps comprend deux demi-corps (**5** et **6**) coulissant l'un dans l'autre, de manière à permettre un réglage de la longueur du corps et des moyens de verrouillage (**16**) du coulissement des demi-corps (**5** et **6**).

## Description

La présente invention concerne le domaine technique des dispositifs destinés à être implantés, soit en vue d'assurer l'ostéosynthèse de deux parties d'os cassés ou, encore, de deux os disjoints suite à un traumatisme, qui doivent être soudés l'un à l'autre, soit en vue de fixer une ostéotomie.

Il est connu, en vue d'assurer une ostéotomie ou ostéosynthèse de deux parties d'os séparées suite à une rupture, de mettre en oeuvre une plaque comprenant un certain nombre de perçages pour la mise en place de vis d'ancrage osseux.

De telles plaques, réalisées en matériau biocompatible, sont fabriquées de manière à présenter différentes formes et dimensions, en fonction des os sur lesquels elles sont susceptibles d'être mises en oeuvre.

Si de telles plaques permettent, effectivement, de couvrir un grand nombre de situations, elles présentent toutefois l'inconvénient d'offrir une gamme discrète de dimensions, de sorte que, dans certaines situations, le chirurgien n'est pas en mesure de trouver une plaque parfaitement adaptée à ses besoins, car la plaque, qui serait exactement correspondante, se trouverait en fait entre deux tailles de plaques qui sont effectivement à sa disposition.

Il est donc apparu le besoin d'un nouveau type de dispositifs d'ostéosynthèse qui soit à-même d'offrir au chirurgien une plus grande souplesse d'utilisation, ainsi qu'une parfaite capacité à s'adapter à la configuration réelle de mise en oeuvre du dispositif d'ostéosynthèse.

Afin d'atteindre cet objectif, l'invention concerne un dispositif implantable d'ostéosynthèse, comprenant un corps allongé pourvu, au moins au niveau de ses deux extrémités, de moyens d'ancrage dans un os ou une partie d'os.

Selon l'invention, ce dispositif d'ostéosynthèse est caractérisé en ce que le corps comprend, d'une part, deux demi-corps coulissant l'un dans l'autre, de manière à permettre, par un réglage de la longueur du corps, un ajustement de la distance entre les moyens d'ancrage des extrémités du corps et, d'autre part, des moyens de verrouillage du coulissement des demi-corps.

La mise en oeuvre des demi-corps coulissants permet ainsi une adaptation continue des dimensions et, plus particulièrement, de la longueur du dispositif d'ostéosynthèse, de manière à assurer une parfaite adéquation de la longueur du dispositif à la dimension et à la géométrie de l'ostéosynthèse que souhaite réaliser le chirurgien.

De manière préférée, le coulissement relatif des deux demi-corps s'effectue dans un mouvement de translation, de sorte que la liaison, réalisée entre les demi-corps peut être, soit du type prismatique, soit du type pivot-glissant, sans que le mouvement de translation relatif des demi-corps soit asservi à un quelconque mouvement de rotation.

Une telle liaison entre les demi-corps peut alors être réalisée de toute façon appropriée.

Selon une forme préférée mais non exclusive de réalisation, l'un des demi-corps, dit femelle, comprend au moins un tube destiné à recevoir une tige de l'autre demi-corps, dit mâle, de manière que la tige coulisse dans le tube du demi-corps femelle.

Le tube et la tige peuvent alors être conformés, soit de manière à permettre une rotation axiale relative des extrémités d'ancrage du dispositif avant verrouillage, soit, au contraire, de manière à empêcher toute rotation axiale relative des extrémités d'ancrage du dispositif avant verrouillage de ce dernier.

Selon l'invention, les moyens de verrouillage peuvent être réalisés de toute façon appropriée.

Ainsi, il pourrait être envisagé de réaliser les moyens de verrouillage sous la forme d'un système de « dents de loup » ou en « sapin », aménagé sur le demi-corps mâle et coopérant avec des conformations complémentaires du demi-corps femelle.

De manière préférée, les moyens de verrouillage sont adaptés pour assurer un verrouillage, par sertissage ou déformation plastique, de l'un des deux demi-corps. Ainsi, dans une forme préférée de réalisation, les moyens de verrouillage comprennent, d'une part, une ou plusieurs aspérités aménagées sur la tige du demi-corps mâle et, d'autre part, le tube du demi-corps femelle qui est plastiquement déformable, de manière à permettre un sertissage du tube sur les aspérités de la tige et assurer alors le verrouillage du coulissement relatif du demi-corps mâle et du demi-corps femelle et donc une liaison complète entre ces derniers.

Un tel mode de réalisation du verrouillage par sertissage, permet de réaliser un dispositif d'ostéosynthèse particulièrement peu encombrant et facile à mettre en oeuvre. Ainsi, le dispositif d'ostéosynthèse selon l'invention se trouve particulièrement adapté pour la réparation des os de la main ou du pied, sans qu'il s'agisse ici d'une application exclusive, étant entendu que le dispositif selon l'invention peut être utilisé pour tout type d'os.

Dans une forme préférée, mais non exclusive de réalisation, les aspérités comprennent une série de gorges annulaires aménagées sur la tige.

Bien entendu, il pourrait être envisagé de réaliser ces aspérités d'une toute autre façon, telle que, par exemple, sous la forme de cannelures ou, encore, d'un filet hélicoïdal, sans que ce dernier coopère avec un filetage du tube du demi-corps femelle dont l'intérieur sera donc lisse.

Selon une caractéristique de l'invention, la tige est conformée de manière à assurer un parfait blocage de la rotation relative des demi-corps après sertissage.

Afin d'atteindre cet objectif, la tige peut alors être, par exemple, conformée de manière à présenter, à la fois, des gorges annulaires et des cannelures longitudinales.

Selon l'invention, les moyens d'ancrage, équipant au moins les extrémités du dispositif et donc les extrémités distales des demi-corps mâle et femelle, peuvent être réalisés de toute façon appropriée.

Selon une caractéristique de l'invention, les moyens d'ancrage comprennent des orifices de réception d'au moins une agrafe d'ancrage osseux ou, encore, d'un clou d'ancrage osseux. Selon une autre caractéristique de l'invention, les moyens d'ancrage comprennent au moins une ouverture de réception d'une vis d'ancrage osseux.

Dans une forme préférée de réalisation, l'ouverture de réception de la vis comprend alors une échancrure pour le passage du corps de la vis, permettant ainsi un dégagement de la vis par rapport à l'ouverture de réception, sans démontage complet de la vis lorsque cette dernière a déjà été engagée dans un os.

Selon une autre caractéristique de l'invention, les moyens d'ancrage comprennent au moins une pointe d'ancrage osseux solidaire du dispositif.

Bien entendu, il est possible de mettre en oeuvre, sur un même et seul dispositif conforme à l'invention, les différentes formes de réalisation des moyens d'ancrage, telles qu'évoquées ci-dessus.

Diverses autres caractéristiques de l'invention ressortent de la description ci-dessous effectuée en références aux dessins annexés qui illustrent différentes formes de réalisation, non limitatives, d'un dispositif d'ostéosynthèse conforme à l'invention.

La **fig. 1** est une perspective d'une première forme de réalisation d'un dispositif d'ostéosynthèse.

La **fig. 2** est une perspective éclatée du dispositif d'ostéosynthèse selon la **fig. 1.**

Les **fig. 3** et **4** sont des perspectives, analogues à la **fig. 1,** montrant d'autres formes de réalisation du dispositif d'ostéosynthèse selon l'invention.

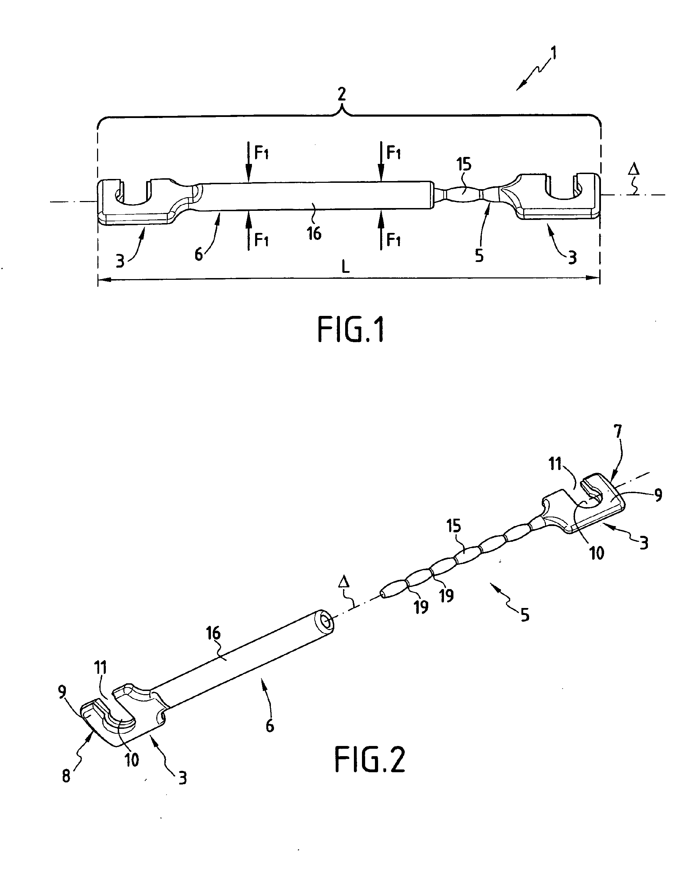

Un dispositif d'ostéosynthèse selon l'invention, tel qu'illustré à la **fig. 1** et désigné dans son ensemble par la référence **1,** comprend un corps **2**, de forme allongée, pourvu, au niveau de ses deux extrémités, de moyens d'ancrage **3** dans un os ou une partie d'os.

Conformément à une caractéristique essentielle de l'invention et comme le montre plus particulièrement la **fig. 2,** le corps **2** comprend deux demi-corps **5** et **6.** Les moyens d'ancrage **3** sont alors aménagés au niveau des extrémités **7**, **8** des demi-corps **5** et **6** qui pourraient être qualifiés de distales.

Selon l'exemple illustré, les extrémités **7** et **8**, respectivement des demi-corps **5** et **6**, sont chacune réalisées sous la forme d'une plaquette **9** comprenant, en tant que moyen d'ancrage, une ouverture **10** destinée à recevoir une vis d'ancrage osseux, non représentée. Selon l'exemple illustré, chaque ouverture **10** comprend une échancrure **11**, destinée à permettre un engagement de la plaquette b9 sur une vis, alors que cette dernière est, en partie au moins, vissée à l'intérieur d'un os. Selon l'exemple illustré, l'échancrure **11** présente une orientation latérale ou transversale par rapport au corps **2.** Toutefois, l'une ou l'autre ou les deux échancrures **11** pourraient présenter une orientation longitudinale et s'ouvrir au niveau des extrémités distales des plaquettes **9**.

Afin de permettre un réglage de la longueur **L** du dispositif **2** et donc un ajustement de la distance entre les moyens d'ancrage **3** et, selon l'exemple illustré, entre les ouvertures **10** de réception d'une vis d'ancrage, l'un des demi-corps **5**, dit mâle, comprend, en tant que moyens de liaison avec l'autre demi-corps, dit femelle **6,** une tige **15**.

Le demi-corps femelle **6** comprend alors un tube **16**, à l'intérieur duquel la tige **1**5 est destinée à venir s'engager et à coulisser.

Afin de permettre un verrouillage du coulissement relatif du demi-corps mâle **5** dans le demi-corps femelle **6** et donc de la tige **15** dans le tube **16**, le dispositif **1** comprend, en tant que moyens de verrouillage, une série de gorges annulaires **19** réalisées à espaces réguliers sur la tige **15**. Selon l'exemple illustré, la série de gorges **19** confère, à la tige **15,** la forme d'une série de grains de riz juxtaposés.

Les gorges annulaires **19**, formant alors des aspérités à la surface de la tige **15**, sont alors mises à profit pour immobiliser la tige **15** dans le tube **16** par déformation du plastique de ce dernier pour réaliser une sorte de sertissage du tube **16** plastiquement déformable autour de la tige **15**. Une fois ce sertissage réalisé, le coulissement relatif du demi-corps mâle **5** par rapport au demi-corps femelle 6 se trouve alors neutralisé.

Le verrouillage par sertissage peut ainsi être effectué en cours de mise en place ou implantation chirurgicale du dispositif d'ostéosynthèse **1** selon l'invention, au moyen d'une pince venant serrer le tube **16** en différents endroits, comme indiqué par les flèches **F**_{**1**}.

Selon l'exemple illustré, la tige **15** présente une forme à symétrie de résolution, d'axe correspondant à l'axe longitudinal Δ du dispositif. Cette symétrie de révolution, associée à la conformation cylindrique de révolution de l'intérieur du tube **16**, permet d'autoriser, en plus de la translation, une rotation angulaire entre les extrémités distales **7** et **8** des demi-corps mâle **5** et femelle **6**.

Selon l'invention, afin de permettre une rotation relative des demi-corps avant verrouillage et garantir un blocage parfait de la translation et de la rotation, après verrouillage par sertissage, il est possible d'adopter, pour la tige **15**, une forme, sans symétrie de révolution, telle qu'une forme prismatique, à section polygonale, tandis que l'intérieur du tube **16** présente une forme cylindrique de révolution.

Ainsi, la combinaison des gorges annulaires et de la section droite polygonale de la tige, avec la forme cylindrique de révolution de l'intérieur du tube, permet, avant sertissage, une translation et une rotation relative des deux corps, tandis qu'après verrouillage il est obtenu une parfaite neutralisation de la rotation et de la translation relative des deux demi-corps.

Toutefois, conformément à l'invention, il pourrait être envisagé de réaliser les moyens de liaison entre le demi-corps mâle **5** et le demi-corps femelle **6**, de manière à empêcher toute rotation avant verrouillage et autoriser un simple coulissement entre les demi-corps.

Ainsi, il pourrait être envisagé de munir le demi-corps mâle **5**, de deux tiges parallèles, destinées à venir s'engager dans deux tubes, également parallèles, équipant le demi-corps femelle **6**. De la même façon, il pourrait être envisagé de conférer au tube **16** et à la tige **15**, une forme prismatique empêchant toute rotation axiale.

Selon l'invention, les demi-corps mâle **5** et femelle **6** peuvent être réalisés en tout matériau approprié biocompatible et, de préférence, en métal, étant entendu que le métal constitutif du demi-corps femelle **6** est choisi de manière à permettre un sertissage du tube **16** autour de la tige **15** au moyen, de préférence, d'une pince à main.

Selon l'exemple décrit précédemment, illustré aux **fig. 1** et **2,** les moyens d'ancrage **3** du dispositif **1** sont constitués sous la forme d'ouvertures **10** de réception de vis.

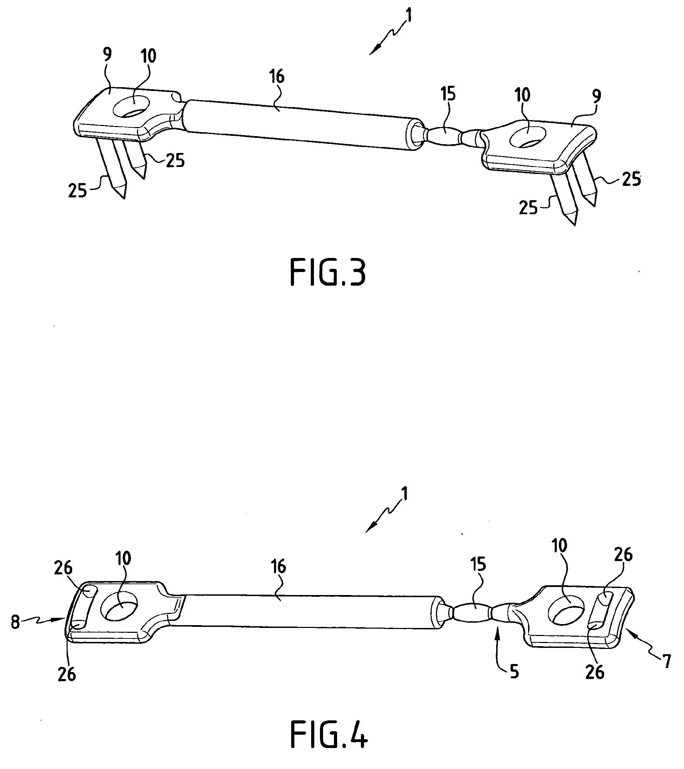

Toutefois, un tel de mode de réalisation des moyens d'ancrage **3** n'est pas exclusif. Ainsi, la **fig. 3** illustre une autre forme de réalisation des moyens d'ancrage **3.**

Selon l'exemple illustré à la **fig**. **3,** les moyens d'ancrage comprennent, en plus d'une ouverture **10** de réception d'une vis d'ancrage osseux, des pointes **25** destinées à être engagées dans un os ou une partie osseuse.

Ainsi selon l'exemple illustré, chaque extrémité distale **8** et **9** comprend deux pointes solidaires de la plaquette **9** correspondante.

Selon l'exemple illustré à la **fig**. **4**, le dispositif d'ostéosynthèse comprend, au niveau de chacune de ses extrémités, en plus d'une ouverture **10** de réception d'une vis d'ancrage osseux, deux orifices de réception **26** d'une agrafe d'ancrage osseux.

Bien entendu, il pourrait également être prévu un seul orifice destiné à recevoir une pointe d'ancrage osseux.

De même, il pourrait être prévu, en tant que moyens d'ancrage osseux, des pointes seules ou, encore, des pointes en association avec des agrafes rapportées ou, encore, des agrafes rapportées, seules ou en association avec des orifices aménagés en vue de leur réception au niveau des extrémités du dispositif **1**.

De même, selon les exemples illustrés **fig. 3** et **4,** les ouvertures **10** de réception de vis d'ancrage présentent une forme circulaire, mais elles pourraient tout aussi bien présenter une forme oblongue.

Par ailleurs, il doit être remarqué que le dispositif selon l'invention peut être utilisé en vue d'exercer un effort de rapprochement entre les parties osseuses à solidariser ou, encore, pour effectuer une distraction de ces deux parties osseuses.

Bien entendu, diverses modifications peuvent être apportées à l'invention sans sortir de son cadre.

## Revendications

1. Dispositif implantable d'ostéosynthèse comprenant un corps allongé pourvu, au moins au niveau de ses deux extrémités, de moyens d'ancrage (**3**) dans un os ou partie d'os,
**caractérisé en ce que** le corps comprend deux demi-corps (**5** et **6**) coulissant l'un dans l'autre, de manière à permettre un réglage de la longueur du corps et des moyens de verrouillage (**19, 16**) du coulissement des demi-corps (**5** et **6**).

2. Dispositif d'ostéosynthèse selon la revendication 1, **caractérisé en ce que** les moyens de verrouillage mettent en oeuvre un sertissage ou une déformation plastique d'au moins un des demi-corps (**5**, **6**).

3. Dispositif d'ostéosynthèse selon la revendication 1 ou 2, **caractérisé en ce que** l'un des demi-corps, dit femelle (**6**) comprend au moins un tube (**16**) destiné à recevoir une tige (**15**) de l'autre demi-corps, dit mâle (**5**), de manière que la tige (**15**) coulisse dans le tube (**16**).

4. Dispositif d'ostéosynthèse selon la revendication 3, **caractérisé en ce que** les moyens de verrouillage comprennent, d'une part, une ou plusieurs aspérités (**19**) aménagées sur la tige (**15**) et, d'autre part, le tube (**16**) du demi-corps femelle (**6**) qui est plastiquement déformable, de manière à permettre un sertissage du tube (**16**) sur les aspérités de la tige (**15**).

5. Dispositif d'ostéosynthèse selon la revendication 4, **caractérisé en ce que** les aspérités (**19**) comprennent une série de gorges annulaires aménagées sur la tige (**15**).

6. Dispositif d'ostéosynthèse selon l'une des revendications 1 à 5, **caractérisé en ce que** la tige (**15**) et le tube (**16**) sont conformés de manière à permettre une rotation axiale relative des extrémités d'ancrage du dispositif avant verrouillage.

7. Dispositif selon la revendication 6, **caractérisé en ce que** la tige est conformée de manière à assurer un blocage parfait de la rotation axiale après verrouillage.

8. Dispositif d'ostéosynthèse selon l'une des revendications 3 à 5, **caractérisé en ce que** la tige (**15**) et le tube (**16**) sont conformés de manière à empêcher toute rotation axiale relative des extrémités d'ancrage avant verrouillage du dispositif.

9. Dispositif d'ostéosynthèse selon l'une des revendications 1 à 8, **caractérisé en ce que** les moyens d'ancrage (**3**) comprennent des orifices (**25**) de réception d'au moins une agrafe ou d'un clou d'ancrage osseux.

10. Dispositif d'ostéosynthèse selon l'une des revendications 1 à 9, **caractérisé en ce que** les moyens d'ancrage (**3**) comprennent au moins une ouverture (**10**) de réception d'une vis d'ancrage osseux.

11. Dispositif d'ostéosynthèse selon l'une des revendications 1 à 10, **caractérisé en ce que** l'ouverture de réception de la vis comprend une échancrure (**11**) pour le passage du corps de la vis.

12. Dispositif d'ostéosynthèse selon l'une des revendications 1 à 11, **caractérisé en ce que** les moyens (**3**) d'ancrage comprennent au moins une pointe (**25**) d'ancrage osseux solidaire du dispositif..
